# EUROPEAN PATENT SPECIFICATION

(11) **EP 1 004 296 B1**
(45) Date of publication and mention of the grant of the patent: **18.08.2004**
(21) Application number: 99921239.2
(22) Date of filing: 24.05.1999
(51) Int. Cl.: A61K 9/14, A61K 9/20, A61K 47/42, A61K 47/04, A61K 47/38, A61K 47/32, A61K 31/355, A61K 47/02

(54) **POWDER CONTAINING FAT-SOLUBLE DRUG**
PUDER MIT FETTLÖSLICHEM WIRKSTOFF
POUDRE CONTENANT UN MEDICAMENT LIPOSOLUBLE

(30) Priority: 26.05.1998 JP 14445198; 19.05.1999 JP 17444699
(43) Date of publication of application: 31.05.2000
(73) Proprietor: Eisai Co., Ltd., Tokyo 112-0002 (JP); FUJI CHEMICAL INDUSTRY CO., LTD., Nakaniikawa-gun, Toyama 930-0397 (JP)
(72) Inventor: KATO, Yoshiteru, Honjo-shi Saitama Prefecture 367-0043 (JP); NAKAMURA, Yukei, Honjo-shi Saitama Prefecture 367-0048 (JP); TATEISHI, Kimio, Honjo-shi Saitama Prefecture 367-0046 (JP); YOKOI, S., Fuji Chemical Industry Co. Ltd., Toyama Prefecture 930-0397 (JP); TANAKA, N., Fuji Chem. Ind. Co., Ltd., Toyama Pref. 930-0397 (JP); HOSOKAWA, T., Fuji Chem. Ind. Co., Ltd, Nakaniikawa-gun Toyama Pref. 930-0397 (JP)
(74) Representative: HOFFMANN - EITLE
(86) International application number: PCT/JP1999/002705
(87) International publication number: WO 1999/061000

(56) References cited:
- JP-A- 1 061 417
- JP-A- 6 461 417
- JP-A- 58 055 412
- US-A- 3 749 799
- US-A- 4 892 889
- US-A- 4 935 245
- US-A- 5 356 636
- PATENT ABSTRACTS OF JAPAN vol. 013, no. 260 (C-607), 15 June 1989 (1989-06-15) & JP 01 061417 A (EISAI CO LTD), 8 March 1989 (1989-03-08)
- PATENT ABSTRACTS OF JAPAN vol. 013, no. 549 (C-662), 7 December 1989 (1989-12-07) & JP 01 226808 A (EISAI CO LTD), 11 September 1989 (1989-09-11)

## Description

The present invention relates to powder containing a fat-soluble drug and having good flowability, and a production process thereof.

A great number of powder containing a fat-soluble vitamin or production processes thereof have been known. For example, U.S. Patent No. 2,756,177 discloses a production process of powder obtained by drying an emulsion containing a vitamin active substance, water, gelatin and/or gum arabic, and a saccharide. Japanese Patent Application Laid-Open No. 59915/1983 discloses a vitamin E-containing powder preparation comprising 50 to 60 wt.% of vitamin E, 0.5 to 2.0 wt.% of silicon dioxide, 1 to 25 wt.% of hydrolyzed gelatin and 20 to 30 wt.% of sodium casein, and Japanese Patent Application Laid-Open No. 200273/1993 discloses a powder product obtained by dispersing a core substance in an aqueous solution of amylose and spraying the dispersion using hydrophobic silica as an auxiliary. Silicon dioxide and hydrophobic silica in these techniques are used for imparting flowability to the respective resulting powders.

On the other hand, Japanese Patent Application Laid-Open No. 501686/1991 (through PCT route) discloses a process for producing free-flowing, spray-dried edible powder containing an edible oil by using hydrolyzed gelatin having a weight average molecular weight of about 15,000 to about 35,000 as measured by gel permeation chromatography.

### PROBLEMS SOUGHT FOR SOLUTION BY THE INVENTION

Such many techniques as described above have been known for obtaining flowable powder containing a fat-soluble drug such as vitamin E. However, there is a demand for development of more improved techniques from the viewpoints of enhancing the content of the fat-soluble drug as high as possible and preventing the exudation of the fat-soluble drug and its sticking to a tableting machine. In addition, hydrolyzed gelatin, amylose and the like are comparatively expensive, and so improvement is required from the viewpoint of cost. The present inventors have carried out an extensive investigation with a view toward solving these problems. As a result, it has been found that the problems can be solved by the following means, thus leading to completion of the present invention.

According to a first aspect of the present invention, a method for producing a flowable powder including a fat-soluble drug comprising the steps of (i) emulsifying and dispersing a mixture comprising a fat-soluble drug, a silicate adsorbent and gelatin and/casein in water; and (ii) spray-drying the resultant dispersion is provided.

The mixture may further comprise a water-soluble polymer.

The optional water-soluble polymer is preferably a cellulose derivative and/or polyvinyl alcohol.

The fat-soluble drug preferably has a melting point of 80°C or less and is preferably a member of the vitamin E family.

The silicate adsorbent is preferably silicon dioxide, calcium silicate, magnesium alumino silicate or magnesium alumino metasilicate or a combination of any of these.

The flowable powder preferably comprises one part by weight of the fat-soluble drug, 0.05-0.5 parts by weight of gelatin and/or casein and 0.1-0.8 parts by weight of the silicate adsorbent. Alternatively, the flowable powder may comprise one part by weight of the fat-soluble drug, 0.05-0.5 parts by weight of gelatin and/or casein, 0.1-0.7 parts by weight of the silicate adsorbent and 0.01-0.1 parts by weight of the water-soluble polymer.

According to a second aspect of the present inventiona flowable powder comprising 1 part by weight of a fat-soluble drug; 0.05-0.5 parts by weight of gelatin and/or casein; and either (A) 0.1-0.8 parts by weight of a silicate adsorbent or (B) 0.1-0.7 parts by weight of a silicate adsorbent and 0.01-0.1 parts by weight of a water-soluble polymer, and being obtainable by emulsifying and dispersing the fat-soluble drug; gelatin and/or casein; and the silicate adsorbent or the silicate adsorbent and the water-soluble polymer in water and spray-drying the resultant dispersion is provided.

The term "fat-soluble drug" as used in the present invention means a physiologically active substance insoluble or hardly soluble in water and relatively easy to dissolve in alcohols such as octanol, oils and fats, , and the like. The melting point of the fat-soluble drug is desirably about 80°C or lower. As examples of the fat-soluble drug, may be mentioned vitamin E family and the like. More specifically, there may be mentioned dl- α -tocopherol, d- α -tocopherol, dl-α -tocopherol acetate, d- α -tocopherol acetate, and besides β, γ and δ homologues thereof, dl-α -tocopherol succinate, d-α -tocopherol succinate, vitamin A oil, tocotrienol, etc. Besides the above, drugs such as teprenone, indomethacin farnesl, tocopherol nicotinate, ubidecarenone, menatetrenone and phytonadione may also be used.

In the present invention, no particular limitation is imposed on gelatin. However, it generally means that produced by treating bone, skin, ligamentum, tendon or the like of an animal with an acid or alkali and extracting the thus-obtained collagen with water under heating. No particular limitation is imposed on the isotonic point, molecular weight, viscosity and the like of gelatin used in the present invention. With respect to gelatin wherein the molecular weight thereof has been controlled to thousands to tens of thousands by hydrolysis, for example, such a gelatin is comparatively expensive, and hence, it has a great effect from an economical point of view to use gelatin which is not subjected to such a treatment.

In the present invention, no particular limitation is imposed on casein. Casein is a phosphoprotein which is a main component of milk, and is not a single substance, but a mixture of similar proteins. It consists of at least three components (α, β and γ), and its molecular weight is about 75,000 to 375,000. In the present invention, the use of sodium casein having good solubility is preferred because of its good handling property.

In the present invention, the silicate adsorbent means powder of a porous silicate. As examples thereof, may be mentioned silicon oxide, calcium silicate, magnesium alumino silicate [Neusilin (trademark) A, product of Fuji Chemical Industry Co., Ltd.], magnesium alumino metasilicate [Neusilin (trademark), product of Fuji Chemical Industry Co., Ltd.], etc. Silicon dioxide, calcium silicate and magnesium alumino metasilicate are preferred. In the present invention, a component having good oil-absorbing ability, such as dextrin or calcium hydrogenphosphate, may be used in combination with the slicate adsorbent if desired.

In the present invention, the water-soluble polymer is generally a cellulose derivative or polyvinyl alcohol. As examples of the cellulose derivative, may be mentioned hydroxypropyl cellulose, hydroxypropyl methylcellulose and sodium carboxymethylcellulose, and the like. With respect to these cellulose derivatives, some kinds of derivatives have been known according to properties such as viscosity. However, no particular limitation is imposed thereon in the present invention. When the water-soluble polymer is used in the present invention, the emulsion stability of the fat-soluble drug, gelatin, casein and adsorbent in water is improved, so that the quality of the resulting flowable powder is enhanced.

Examples of cellulose and/or cellulose derivatives include those that are soluble in water or dissolve in water to become viscous liquids. Examples thereof include methylcellulose, hydroxypropyl cellulose [for example, Nisso HPC, trade name: product of Nippon Soda Co., Ltd.; and Shinetsu HPC, trade name; product of Shi-Etsu Chemical Co., Ltd.], hydroxypropyl methylcellulose-2208, -2906 and - 2910 [for example, Metholose 90SH, 65SH and 60SH, trade names: products of Shi-Etsu Chemical Co., Ltd.; and Methocel K, F and E, trade names, and Marbolose, trade name TC-51: products of Dow Chemical Co., Ltd.], and hydroxyethyl cellulose, and the like.

As cellulose and/or cellulose derivatives that are insoluble in water or partially dissolve in or swell with water, there may also be used, for example, crystalline cellulose, sodium crystalline cellulose · carmelose, ethyl cellulose, hydroxypropyl cellulose having a low degree of substitution, hydroxypropyl methylcellulose phthalate, hydroxypropyl methylcellulose acetate succinate, carmelose, calcium carmellose, sodium carmellose, sodium crosscarmellose, carboxymethylethyl cellulose and cellulose acetate phthalate, and the like.

The proportions of the fat-soluble drug, gelatin and/or casein, and silicate adsorbent in the present invention are generally 0.05 to 0.5 parts by weight for gelatin and/or casein, and 0.1 to 0.8 parts by weight for the silicate adsorbent, per part by weight of the fat-soluble drug, and preferably 0.05 to 0.25 parts by weight for gelatin and/or casein, and 0.2 to 0.7 parts by weight for the slicate adsorbent, per part by weight of the fat-soluble drug. When the water-soluble polymer is further used in the present invention, the proportions of these components are generally 0.05 to 0.5 parts by weight for gelatin and/or casein, 0.1 to 0.7 parts by weight for the silicate adsorbent, and 0.01 to 0.1 parts by weight for the water-soluble polymer, per part by weight of the fat-soluble drug, and preferably 0.05 to 0.25 parts by weight for gelatin and/or casein, 0.2 to 0.7 parts by weight for the silicate adsorbent, and 0.005 to 0.1 parts by weight, more preferably 0.015 to 0.1 parts by weight for the water-soluble polymer, per part by weight of the fat-soluble drug.

No particular limitation is imposed on the production process of the flowable powder in the present invention. The flowable powder can be produced, for example, in accordance with the following process. However, for convenience' sake, description will be given as to a case where gelatin is used.

Gelatin is dissolved in purified water heated to about 50 to 60°C, and a water-soluble polymer such as hydroxypropyl methylcellulose is further added and dissolved if circumstances require. Vitamin E (dl-α-tocopherol acetate) is then added to the solution, and the resultant mixture is stirred and emulsified for about 15 minutes by means of a high-speed stirring machine, for example, a Homomixer (trade name) or the like. Additional water heated to about 50 to about 60°C is added as needed, and an adsorbent such as calcium silicate is added to the solution to stir the resultant mixture for about 15 minutes, thereby obtaining a homogeneous suspension. The viscosity of the thus-obtained suspension is generally 20 to 1,000 CP, preferably 30 to 1,000 CP. The suspension is spray-dried by a spray dryer, whereby flowable powder containing vitamin E can be obtained. As the spray dryer, may be used any machine of the disk type and the nozzle type. Conditions of the spray drying are those generally used. For example, the inlet temperature is 210°C, and the outlet temperature is 130°C.

The particle diameter of the flowable powder obtained by the present invention is generally 0.05 to 0.5 mm, and the angle of repose thereof is generally 30 to 40°, so that the powder has good flowability.

When the melting point of the fat-soluble drug used is high, for example, d-α-tocopherol succinate having a melting point of about 75°C is used, a homogeneous suspension can be obtained by raising the temperature at the time the solution is prepared to about 80°C to conduct stirring. In this case, the suspension must not be always heated upon spraying once the homogeneous suspension is prepared.

### EFFECTS OF THE INVENTION

According to the present invention, there can be produced flowable powder containing a fat-soluble drug at a high content of 50% or higher. The flowable powder according to the present invention can be prepared by a direct tableting process into tablets without causing the exudation of the fat-soluble drug and its sticking to a tableting machine by mixing it with an excipient such as a saccharide, or cellulose or a derivative thereof. A marked effect of the present invention is that stain or smear on a spray dryer is extremely slight. When powder containing a fat-soluble drug has heretofore been produced, cleaning has been extremely troublesome because the drug and the like have stuck to the spray dryer. However, cleaning and the like are extremely easy because the drug and the like scarcely stick after the powder according to the present invention is produced.

According to the present invention, there could be provided vitamin E-containing powder which scarcely undergoes the exudation and decomposition of the vitamin E even when the powder is stored for a long period of time, and hence has high shelf stability; the vitamin E is not degraded upon the production thereof

The vitamin E-containing powder according to the present invention has been powder which is free from the exudation of a vitamin E component out of the surface of the powder, is dry, undergoes no aggregation among particles and is easy to store, quantify and transport. According to the present invention, a recovery rate is markedly improved because the powder does not stick to the inner wall of a spry dryer upon the production thereof. In addition, since the spray drying treatment can be conducted in a temperature range comparatively higher than that in the conventional processes, the time required for the drying treatment is shortened, and moreover the time during which the powder is exposed to heat is shortened. Therefore, the production process of the present invention is extremely efficient.

The vitamin E-containing powder according to the present invention is useful as an antioxidant and stabilizer for food, and a medicament.

### EXAMPLES

The present invention will hereinafter be described in more detail by the following Examples. However, the present invention is not limited to these examples.

### Example 1:

Dissolved in 200 g of purified water heated to 50 °C were 10 g of gelatin, and 120 g of dl-α-tocopherol acetate were added to the solution. The resultant mixture was stirred for 10 minutes by a Homomixer (trade name). Further, 200 g of purified water heated to about 50°C were added to the mixture, followed by stirring for 10 minutes by the Homomixer. To the resultant mixture were added 70 g of calcium silicate (product of Tokuyama Soda Co., Ltd., trade name: Florite RE), and the resultant mixture was stirred for 15 minutes by the Homomixer to obtain a homogeneous suspension. The viscosity of the suspension was 150 cp. The suspension was spray-dried under the following conditions to obtain 70 g of flowable powder. The resultant powder had good flowability, a particle diameter of 0.07 mm and a dl-α-tocopherol acetate content of 590 mg per gram of the powder.
Spray drying conditions: inlet temperature 200°C , outlet temperature 100°C , disk revolution speed 30,000 rpm, and spray speed 1.3 L/hr.

### Example 2:

Dissolved in 200 g of purified water heated to 50°C were 20 g of gelatin, and 120 g of dl-α-tocopherol acetate were added to the solution. The resultant mixture was stirred for 10 minutes by a Homomixer (trade name). Further, 200 g of purified water heated to about 50°C were added to the mixture, followed by stirring for 10 minutes by the Homomixer. To the resultant mixture were added 30 g of calcium silicate (product of Tokuyama Soda Co., Ltd., trade name: Florite RE), and the resultant mixture was stirred for 15 minutes by the Homomixer to obtain a homogeneous suspension. The viscosity of the suspension was 300 cp. The suspension was spray-dried under the following conditions to obtain 60 g of flowable powder. The resultant powder had good flowability, a particle diameter of 0.07 mm and a dl-α-tocopherol acetate content of 700 mg per gram of the powder.
Spray drying conditions: inlet temperature 200 °C, outlet temperature 100°C, disk revolution speed 30,000 rpm, and spray speed 1.5 L/hr.

### Example 3:

Dissolved in 260 g of purified water heated to 50°C were 20 g of gelatin and 10 g of hydroxypropyl methylcellulose (product of Shin-Etsu Chemical Co., Ltd., trade name: TC-5E), and 120 g of dl-α-tocopherol acetate were added to the solution. The resultant mixture was stirred for 10 minutes by a Homomixer (trade name). Further, 200 g of purified water heated to about 50°C were added to the mixture, followed by stirring for 10 minutes by the Homomixer. To the resultant mixture were added 30 g of calcium silicate (product of Tokuyama Soda Co., Ltd., trade name: Florite RE), and the resultant mixture was stirred for 15 minutes by the Homomixer to obtain a homogeneous suspension. The viscosity of the suspension was 300 cp. The suspension was spray-dried under the following conditions to obtain 120 g of flowable powder. The resultant powder had good flowability, a particle diameter of 0.07 mm and a dl-α-tocopherol acetate content of 660 mg per gram of the powder.
Spray drying conditions: inlet temperature 200°C, outlet temperature 100°C, disk revolution speed 30,000 rpm, and spray speed 1.5 L/hr.

### Example 4:

Flowable powder was obtained in the same manner as in Example 3 except that 120 g of dl-α-tocopherol acetate were changed to 120 g of d-α-tocopherol acetate. The viscosity of the suspension was 350 cp. The suspension was spray-dried under the following conditions to obtain 110 g of flowable powder. The resultant powder had good flowability, a particle diameter of 0.07 mm and a dl-α-tocopherol acetate content of 658 mg per gram of the powder.
Spray drying conditions: inlet temperature 200°C, outlet temperature 100°C, disk revolution speed 30,000 rpm, and spray speed 1.5 L/hr.

### Example 5:

Dissolved in 260 g of purified water heated to 50°C were 30 g of gelatin and 10 g of hydroxypropyl methylcellulose (product of Shin-Etsu Chemical Co., Ltd., trade name: TC-5E), and 120 g of d-α-tocopherol acetate were added to the solution. The resultant mixture was stirred for 10 minutes by a Homomixer (trade name). Further, 300 g of purified water heated to about 50°C were added to the mixture, followed by stirring for 10 minutes by the Homomixer. To the resultant mixture were added 35 g of calcium silicate (product of Tokuyama Soda Co., Ltd., trade name: Florite RE), and the resultant mixture was stirred for 15 minutes by the Homomixer to obtain a homogeneous suspension. The viscosity of the suspension was 400 cp. The suspension was spray-dried under the following conditions to obtain 120 g of flowable powder. The resultant powder had good flowability, a particle diameter of 0.07 mm and a d-α-tocopherol acetate content of 610 mg per gram of the powder.
Spray drying conditions: inlet temperature 200°C, outlet temperature 100°C, disk revolution speed 30,000 rpm, and spray speed 1.5 L/hr.

### Example 6:

Flowable powder was obtained in the same manner as in Example 1 except that calcium silicate was changed to anhydrous silicic acid (trade name: Sylysia). The viscosity of the suspension was 130 cp. The suspension was spray-dried under the following conditions to obtain 50 g of flowable powder. The resultant powder had good flowability, a particle diameter of 0.06 mm and a dl-α-tocopherol acetate content of 590 mg per gram of the powder.
Spray drying conditions: inlet temperature 200°C, outlet temperature 100°C, disk revolution speed 30,000 rpm, and spray speed 1.3 L/hr.

### Example 7:

Flowable powder was obtained in the same manner as in Example 3 except that 10 g of hydroxypropylmethyl cellulose (trade name: TC-5E) were changed to 2 g of hydroxypropylmethyl cellulose (trade name: TC-5R). The viscosity of the suspension was 200 cp. The suspension was spray-dried under the following conditions to obtain 115 g of flowable powder. The resultant powder had good flowability, a particle diameter of 0.07 mm and a dl-α-tocopherol acetate content of 698 mg per gram of the powder.
Spray drying conditions: inlet temperature 200°C, outlet temperature 100°C, disk revolution speed 30,000 rpm, and spray speed 1.5 L/hr.

### Example 8:

Flowable powder was obtained in the same manner as in Example 3 except that 10 g of hydroxypropylmethyl cellulose (trade name: TC-5E) were changed to 1 g of polyvinyl alcohol. The viscosity of the suspension was 200 cp. The suspension was spray-dried under the following conditions to obtain 85 g of flowable powder. The resultant powder had good flowability, a particle diameter of 0.06 mm and a dl-α-tocopherol acetate content of 690 mg per gram of the powder.
Spray drying conditions: inlet temperature 200°C, outlet temperature 100°C, disk revolution speed 30,000 rpm, and spray speed 1.5 L/hr.

### Example 9:

Dissolved in 62 kg g of purified water heated to 50°C were 5.0 kg of gelatin and 2.5 kg of hydroxypropyl methylcellulose (product of Shin-Etsu Chemical Co., Ltd., trade name: TC-5R), and 30.0 kg of dl-α-tocopherol acetate were added to the solution. The resultant mixture was stirred for 10 minutes by a Homomixer (trade name). Further, 55 kg of purified water heated to about 50°C were added to the mixture, followed by stirring for 15 minutes by the Homomixer. To the resultant mixture were added 7.5 kg of calcium silicate (product of Tokuyama Soda Co., Ltd., trade name: Florite RE), and the resultant mixture was stirred for 15 minutes by the Homomixer to obtain a homogeneous suspension. The viscosity of the suspension was 150 cp. The suspension was spray-dried by a spray dryer of the nozzle type having a water-evaporating capability of 250 kg/hr under the following conditions to obtain 40 kg of flowable powder. No spray trouble occurred during the operation, and the inner wall of the spray dryer after the spray drying was neither stuck nor stained with the sprayed solution, powder and the like, and can be easily cleaned with water, and so operability was extremely excellent. The resultant powder had good flowability, an angle of repose of 35°, a particle diameter of 0.19 mm and a dl-α-tocopherol acetate content of 661 mg per gram of the powder.
Spray drying conditions: inlet temperature 210°C, outlet temperature 130°C, nozzle bore 1.0 mm, and spray speed 100 kg/hr.

### Example 10:

A process was carried out in the same manner as in Example 9 except that 2.5 kg of hydroxypropyl methylcellulose (trade name: TC-5E) were used in place of 2.5 kg of hydroxypropyl methylcellulose (trade name: TC-5R), and 30.0 kg of d-α-tocopherol acetate were used in place of 30.0 kg of dl-α-tocopherol acetate. The viscosity of the suspension was 90 cp. The suspension was spray-dried by a spray dryer of the nozzle type having a water-evaporating capability of 250 kg/hr under the following conditions to obtain 42 kg of flowable powder. No spray trouble occurred during the operation, and the inner wall of the spray dryer after the spray drying was neither stuck nor stained with the sprayed solution, powder and the like, and can be easily cleaned with water, and so operability was extremely excellent. The resultant powder had good flowability, an angle of repose of 34°, a particle diameter of 0.17 mm and a d-α-tocopherol acetate content of 659 mg per gram of the powder.
Spray drying conditions: inlet temperature 210°C, outlet temperature 130°C, nozzle bore 1.0 mm, and spray speed 100 kg/hr.

### Example 11:

Dissolved in 200 g of purified water heated to 50°C were 24 g of gelatin and 1.2 g of sodium carboxymethyl cellulose (the name of a maker: Shin-Etsu Chemical Co., Ltd.), and 120 g of dl-α-tocopherol acetate (vitamin E) were added to the solution. The resultant mixture was stirred for 10 minutes by a homomixer (manufactured by Tokushu Kika Kogyo Co., Ltd., trade name: T.K Homomixer). Further, 250 g of purified water heated to about 50°C were added to the mixture, followed by stirring for 10 minutes by the homomixer. To the resultant mixture were added 34.8 g of calcium silicate, and the resultant mixture was stirred for 15 minutes by the homomixer to obtain a homogeneous suspension. The viscosity of the suspension was 108 cp. The suspension was spray-dried under the following conditions to obtain 116 g of flowable powder having a vitamin E content of 66.7%. The resultant powder had good flowability.
Spray drying conditions: inlet temperature 210°C, outlet temperature 105°C, disk revolution speed 30,000 rpm, and spray speed 2.0 L/hr.

### Example 12:

Dissolved in 200 g of purified water heated to 50°C were 24 g of gelatin and 1.2 g of sodium carboxymethyl cellulose (the name of a maker: Daicel Chemical Industries, Ltd.), and 120 g of dl-α-tocopherol acetate (vitamin E) were added to the solution. The resultant mixture was stirred for 10 minutes by a Homomixer (trade name). Further, 250 g of purified water heated to about 50°C were added to the mixture, followed by stirring for 10 minutes by the Homomixer. To the resultant mixture were added 30 g of calcium silicate and 4.8 g of dextrin, and the resultant mixture was stirred for 15 minutes by the homomixer to obtain a homogeneous suspension. The viscosity of the suspension was 100 cp. The suspension was spray-dried under the following conditions to obtain 128 g of flowable powder having a vitamin E content of 66.7%. The resultant powder had good flowability. The bulk density of the powder was 3.24 ml/g.
Spray drying conditions: inlet temperature 220°C, outlet temperature 105°C, disk revolution speed 30,000 rpm, and spray speed 2.0 L/hr.

### Example 13:

Dissolved in 180 g of purified water heated to 50°C were 21 g of gelatin and 3.0 g of sodium carboxymethyl cellulose (the name of a maker: Daicel Chemical Industries, Ltd.), and 126 g of dl-α-tocopherol acetate (vitamin E) were added to the solution. The resultant mixture was stirred for 10 minutes by a Homomixer (trade name). Further, 250 g of purified water heated to about 50°C were added to the mixture, followed by stirring for 10 minutes by the Homomixer. To the resultant mixture were added 30 g of calcium silicate, and the resultant mixture was stirred for 15 minutes by the homomixer to obtain a homogeneous suspension. The viscosity of the suspension was 129 cp. The suspension was spray-dried under the following conditions to obtain 120 g of flowable powder having a vitamin E content of 70%. The resultant powder had good flowability. The bulk density of the powder was 3.5 ml/g.
Spray drying conditions: inlet temperature 220°C. outlet temperature 105°C, disk revolution speed 30,000 rpm, and spray speed 2.0 L/hr.

### Example 14:

Dissolved in 180 g of purified water heated to 50°C were 21 g of gelatin and 3.0 g of sodium carboxymethyl cellulose (the name of a maker: Daicel Chemical Industries, Ltd.), and 126 g of dl-α-tocopherol acetate (vitamin E) were added to the solution. The resultant mixture was stirred for 10 minutes by a Homomixer (trade name). Further, 250 g of purified water heated to about 50°C were added to the mixture, followed by stirring for 10 minutes by the Homomixer. To the resultant mixture were added 30 g of calcium silicate, and the resultant mixture was stirred for 15 minutes by the homomixer to obtain a homogeneous suspension. The viscosity of the suspension was 129 cp. The suspension was spray-dried under the following conditions to obtain 143 g (yield: 79.5%) of flowable powder having a vitamin E content of 72.5%. The resultant powder had good flowability. The bulk density of the powder was 3.27 ml/g.
Spray drying conditions: inlet temperature 220°C, outlet temperature 105°C, disk revolution speed 30,000 rpm, and spray speed 2.0 L/hr.

### Example 15:

Dissolved in 150 g of purified water heated to 50°C were 20 g of gelatin and 2.0 g of sodium carboxymethyl cellulose (the name of a maker: Daicel Chemical Industries, Ltd.), and 100 g of dl-α-tocopherol acetate (vitamin E) were added to the solution. The resultant mixture was stirred for 10 minutes by a Homomixer (trade name). Further, 1.650 g of purified water heated to about 50°C were added to the mixture, followed by stirring for 10 minutes by the Homomixer. To the resultant mixture were added 80 g of anhydrous silicic acid (Aerosil 389), and the resultant mixture was stirred for 15 minutes by the homomixer to obtain a homogeneous suspension. The viscosity of the suspension was 40 cp. The suspension was spray-dried under the following conditions to obtain 118 g of flowable powder having a vitamin E content of 50%. The resultant powder had good flowability. The bulk density of the powder was 2.70 ml/g.
Spray drying conditions: inlet temperature 220°C, outlet temperature 105°C, disk revolution speed 30,000 rpm, and spray speed 2.0 L/hr.

### Example 16:

Dissolved in 160 g of purified water heated to 50°C were 20 g of casein and 2.0 g of sodium carboxymethyl cellulose (the name of a maker: Daicel Chemical Industries, Ltd.), and 80 g of dl-α-tocopherol acetate (vitamin E) were added to the solution. The resultant mixture was stirred for 10 minutes by a Homomixer (trade name). Further, 300 g of purified water heated to about 50°C were added to the mixture, followed by stirring for 10 minutes by the Homomixer. To the resultant mixture were added 58 g of anhydrous silicic acid (Aerosil 380, trade name), and the resultant mixture was stirred for 15 minutes by the homomixer to obtain a homogeneous suspension. The viscosity of the suspension was 128 cp. The suspension was spray-dried under the following conditions to obtain 116.6 g (yield: 72.9%) of flowable powder having a vitamin E content of 50%. The resultant powder had good flowability. The bulk density of the powder was 2.48 ml/g.
Spray drying conditions: inlet temperature 220°C, outlet temperature 105°C, disk revolution speed 30,000 rpm, and spray speed 1.5 L/hr.

### Example 17:

Dissolved in 60 L of water were 2.5 kg of hydroxypropyl methylcellulose and 5.0 kg of gelatin at about 50°C. To this solution were added 30 kg of dl-α-tocopherol acetate (vitamin E) under stirring, and the resultant mixture was subjected to an emulsifying treatment by a Homomixer (trade name). Then, 45 L of water were added to the resultant emulsion, and the resultant mixture was subjected again to the emulsifying treatment. To the resultant suspension were added 7.5 kg of porous powder of calcium silicate (product of Tokuyama Soda Co., Ltd., trade name: Florite RE) with stirring. The resultant suspension was spray-dried under conditions of an inlet temperature of 230°C, an outlet temperature of 135°C to 140°C and a feed flow rate of 105 kg/hr to obtain 37.2 kg (yield: 82.6%) of white flowable powder having a vitamin E content of 67%.

The particle diameter distribution of the resultant powder was as follows: 60 mesh (hereinafter referred to as M) (15.2%), 100 M (64.1%) and 200 M (18.1%), and no aggregation among powder particles was observed.

### Example 18:

Dissolved in 60 L of water were 2.5 kg of hydroxypropyl methylcellulose and 5.0 kg of gelatin at about 50°C. To this solution were added 30 kg of dl-α-tocopherol acetate (vitamin E) under stirring, and the resultant mixture was subjected to an emulsifying treatment by a Homomixer (trade name). Then, 50 L of water were added to the resultant emulsion, and the resultant mixture was subjected again to the emulsifying treatment. To the resultant suspension were added 7.5 kg of Florite RE with stirring. The resultant suspension was spray-dried under conditions of an inlet temperature of 211°C to 219°C, an outlet temperature of 132°C, to 134°C and a feed flow rate of 95 to 105 kg/hr to obtain 36.4 kg (yield: 80.9%) of white flowable powder having a vitamin E content of 67%.

The particle diameter distribution of the resultant powder was as follows: 60 M (24.5%), 100 M (56.3%) and 200 M (16.3%), and no aggregation among powder particles was observed.

### Example 19:

A process was carried out in the same manner as in Example 16 except that 10 g of casein and 10 g of gelatin were used in place of 20 g of casein. The results thereof were as follows:

153 g (yield: 76.5%) of flowable powder having a vitamin E content of 50%; bulk density of the powder: 2.35 ml/g.

### Example 20:

A process was carried out in the same manner as in Example 1 except that 120 g of teprenone were used in place of 120 g of dl-α-tocopherol acetate. The results thereof were as follows:
136 g (yield: 68%) of flowable powder having a teprenone content of 60%; bulk density of the powder: 4.2 ml/g.

## Claims

1. A method for producing a flowable powder including a fat-soluble drug comprising the steps of:
(i) emulsifying and dispersing a mixture comprising a fat-soluble drug, a silicate adsorbent and gelatin and/or casein in water; and
(ii) spray-drying the resultant dispersion.

2. A method according to Claim 1, wherein the mixture further comprises a water-soluble polymer.

3. A method according to Claim 2, wherein the water-soluble polymer is a cellulose derivative and/or polyvinyl alcohol.

4. A method according to any preceding Claim, wherein the fat-soluble drug has a melting point of 80°C or less.

5. A method according to any preceding Claim, wherein the fat-soluble drug is a member of the vitamin E family.

6. A method according to any preceding Claim, wherein the silicate adsorbent is silicon dioxide, calcium silicate, magnesium alumino silicate or magnesium alumino metasilicate or a combination of any of these.

7. A method according to any preceding Claim, wherein the flowable powder comprises 1 part by weight of the fat-soluble drug, 0.05 - 0.5 parts by weight of gelatin and/or casein and 0.1 - 0.8 parts by weight of the silicate adsorbent.

8. A method according to Claim 2 or Claim 3, wherein the flowable powder comprises 1 part by weight of the fat-soluble drug, 0.05 - 0.5 parts by weight of gelatin and/or casein, 0.1 - 0.7 parts by weight of the silicate adsorbent and 0.01 - 0.1 parts by weight of the water-soluble polymer.

9. A flowable powder comprising:
1 part by weight of a fat-soluble drug;
0.05 to 0.5 parts by weight of gelatin and/or casein; and
either (A) 0.1 to 0.8 parts by weight of a silicate adsorbent or (B) 0.1 to 0.7 parts by weight of a silicate adsorbent and 0.01 to 0.1 parts by weight of a water-soluble polymer,
and being obtainable by emulsifying and dispersing
the fat-soluble drug;
gelatin and/or casein, and
the silicate adsorbent or the silicate adsorbent and the water-soluble polymer, in water and spray-drying the resultant dispersion.

## Patentansprüche

1. Verfahren zur Herstellung eines fliessfähigen Pulvers, das ein fettlösliches Arzneimittel einschliesst, welches die folgenden Schritte umfasst:
(i) Emulgieren und Dispergieren einer Mischung, die ein fettlösliches Arzneimittel, ein Silicat-Adsorptionsmittel und Gelatine und/oder Casein in Wasser enthält; und
(ii) Sprühtrocknen der erhaltenen Dispersion.

2. Verfahren gemäss Anspruch 1, wobei die Mischung ferner ein wasserlösliches Polymer enthält.

3. Verfahren gemäss Anspruch 2, wobei das wasserlösliche Polymer ein Cellulosederivat und/oder Polyvinylalkohol ist.

4. Verfahren gemäss mindestens einem der vorstehenden Ansprüche, wobei das fettlösliche Arzneimittel einen Schmelzpunkt von 80°C oder weniger hat.

5. Verfahren gemäss mindestens einem der vorstehenden Ansprüche, wobei das fettlösliche Arzneimittel ein Vertreter der Vitamin E-Familie ist.

6. Verfahren gemäss mindestens einem der vorstehenden Ansprüche, wobei das Silicat-Adsorptionsmittel Siliciumdioxid, Calciumsilicat, Magnesiumaluminosilicat oder Magnesiumaluminometasilicat oder eine beliebige Kombination davon ist.

7. Verfahren gemäss mindestens einem der vorstehenden Ansprüche, wobei das fliessfähige Pulver 1 Gew.-Teil des fettlöslichen Arzneimittels, 0,05 bis 0,5 Gew.-Teile Gelatine und/oder Casein und 0,1 bis 0,8 Gew.-Teile des Silicat-Adsorptionsmittels enthält.

8. Verfahren gemäss Anspruch 2 oder 3, wobei das fliessfähige Pulver 1 Gew.-Teil des fettlöslichen Arzneimittels, 0,05 bis 0,5 Gew.-Teile Gelatine und/oder Casein, 0,1 bis 0,7 Gew.-Teile des Silicat-Adsorptionsmittels und 0,01 bis 0,1 Gew.-Teile des wasserlöslichen Polymers enthält.

9. Fliessfähiges Pulver, das umfasst:
1 Gew.-Teil eines fettlöslichen Arzneimittels;
0,05 bis 0,5 Gew.-Teile Gelatine und/oder Casein; und
entweder (A) 0,1 bis 0,8 Gew.-Teile eines Silicat-Adsorptionsmittels oder (B) 0,1 bis 0, 7 Gew. -Teile eines Silicat-Adsorptionsmittels und 0,01 bis 0,1 Gew.-Teile eines wasserlöslichen Polymers,
und das erhältlich ist durch Emulgieren und Dispergieren
des fettlöslichen Arzneimittels;
von Gelatine und/oder Casein; und
des Silicat-Adsorptionsmittels oder des Silicat-Adsorptionsmittels und des wasserlöslichen Polymers in Wasser und Sprühtrocknen der erhaltenen Dispersion.

## Revendications

1. Procédé pour la production d'une poudre susceptible de s'écouler incluant un médicament liposoluble comprenant les étapes de :
(i) émulsifier et mettre en dispersion un mélange comprenant un médicament liposoluble, un adsorbant en silicate et de la gélatine et/ou de la caséine dans de l'eau ; et
(ii) sécher par atomisation la dispersion résultante.

2. Procédé selon la revendication 1, dans lequel le mélange comprend en plus un polymère soluble dans l'eau.

3. Procédé selon la revendication 2, dans lequel le polymère soluble dans l'eau est un dérivé de cellulose et/ou un alcool de polyvinyle.

4. Procédé selon l'une quelconque des revendications précédentes, dans lequel le médicament liposoluble a un point de fusion de 80 °C ou moins.

5. Procédé selon l'une quelconque des revendications précédentes, dans lequel le médicament liposoluble est un membre de la famille de la vitamine E.

6. Procédé selon l'une quelconque des revendications précédentes, dans lequel l'adsorbant en silicate est du dioxyde de silicium, du silicate de calcium, du silicate de magnésium alumino ou du métasilicate de magnésium alumino ou une combinaison de n'importe lequel de ceux-ci.

7. Procédé selon l'une quelconque des revendications précédentes, dans lequel la poudre susceptible de s'écouler comprend 1 partie en poids du médicament liposoluble, 0,05 - 0,5 partie en poids de gélatine et/ou de caséine et 0,1 - 0,8 partie en poids de l'adsorbant en silicate.

8. Procédé selon la revendication 2 ou la revendication 3, dans lequel la poudre susceptible de s'écouler comprend 1 partie en poids du médicament liposoluble, 0,05 - 0,5 partie en poids de gélatine et/ou de caséine et 0,1 - 0,7 partie en poids de l'adsorbant en silicate et 0,01 - 0,1 partie en poids du polymère soluble dans l'eau.

9. Poudre susceptible de s'écouler comprenant :
1 partie en poids du médicament liposoluble ;
0,05 - 0,5 partie en poids de gélatine et/ou de caséine ;
soit (A) 0,1 - 0,8 partie en poids de l'adsorbant en silicate soit (B) 0,1 - 0,7 partie en poids de l'adsorbant en silicate et 0,01 - 0,1 partie en poids du polymère soluble dans l'eau,
et étant susceptible d'être obtenu en émulsifiant et en mettant en dispersion :
le médicament soluble dans l'eau ;
de la gélatine et/ou de la caséine ; et
l'adsorbant en silicate ou l'adsorbant en silicate et le polymère soluble dans l'eau, dans de l'eau et en séchant par atomisation la dispersion résultante.
